Europäisches Patentamt

European Patent Office

Office européen des brevets

(19)

(11) Publication number: **0 249 475 A2**

# EUROPEAN PATENT APPLICATION

(12)

(21) Application number: **87305171.8**

(22) Date of filing: **11.06.87**

(51) Int. Cl.4: **A 61 M 37/00**

(30) Priority: **13.06.86 US 874262**

(43) Date of publication of application:
**16.12.87 Bulletin 87/51**

(84) Designated Contracting States:
**AT BE CH DE ES FR GB GR IT LI NL SE**

(71) Applicant: **ALZA CORPORATION**
**950 Page Mill Road**
**Palo Alto, California 94304 (US)**

(72) Inventor: **Urquhart, John**
**975 Hamilton Avenue**
**Palo Alto California 94301 (US)**

**Libicki, Shari**
**751 B Loma Verde Avenue**
**Palo Alto California 94303 (US)**

(74) Representative: **Evans, David Charles et al**
**F.J. CLEVELAND & COMPANY 40-43, Chancery Lane**
**London, WC2A 1JQ (GB)**

(54) **Delayed percutaneous delivery.**

(57) A device for delivering therapeutic agent to a biological environment by diffusion has at least two members, one of which contains the agent to be delivered, and the other, delay member, being substantially devoid of agent. The members are combined just prior to installation, whereupon agent diffuses from the agent containing member into the agent-free member which is in contact with the environment. The agent-free member absorbs agent, thus delaying the time at which agent enters the environment at therapeutic rates. As a result, dosage administration profiles are obtained whereby one such two-member device is installed at regular intervals, wherein dosing occurs during only a portion of the time during which the device is installed.

**Description**

"DELAYED PERCUTANEOUS DELIVERY"

FIELD OF THE INVENTION

The invention relates to agent delivery devices and more particularly to devices which deliver agent by diffusion from a reservoir for an extended period of time. (As used herein, the terms,"drug" and, "agent" are interchangeable and are intended to have their broadest interpretation as meaning any substance which is administered to a living organism to produce an intended, usually beneficial, effect.)

BACKGROUND OF THE INVENTION

In the administration of therapeutic agents or drugs, it is desired to provide for optimum effects while using a minimum of agent. Delivery devices which deliver agents at controlled rates over extended time periods are capable of providing beneficial effects with the fewest or least severe side-effects. A problem remains, however, where continuous presence of the agent in blood and other body fluids and tissues leads to a decline in therapeutic effect, known as tolerance.

It has long been recognized that tolerance occurs with certain agents. The elimination or reduction of tolerance is typically addressed by increasing the dosage, or by spacing agent dosing so as to provide time intervals during which agent concentrations in blood and other body fluids and tissues fall to zero or below a critical value. To achieve such time intervals where the agent is administered in the form of an oral or injected dosage, the intervals between doses may be extended. This dosage scheme has two drawbacks: (a) where the patient must self-administer the agent, failure to follow the correct regimen often occurs, and (b) where a health service organization must administer the agent, added costs are incurred as a result of the additional attention which must be rendered to maintain the regimen. Moreover, the optimal time for agent administration may occur at inconvenient times, e.g. during the usual hours of sleep.

An example of tolerance is found in the administration of organic nitrates, used to treat angina pectoris and other types of heart disease. For a complete discussion, reference may be had to the Am. Heart Assoc. Abs.: 58th Scientific Sessions, Part II, Vol. 72:4 (1985); Sullivan et.al.: Ineffectiveness of Transdermal Nitroglycerin, JACC Vol. 5:5 (1985); and Am J Cardiol, Vol. 54:6 (1984); Parker, J. et al: Transdermal isosorbide dinitrate in angina pectoris: effect of acute and sustained therapy. Am J Cardiol 54:8-13 (1984); Shell, W.: Mechanisms and Therapy of Silent Myocardial Ischemia and the effect of transdermal nitroglycerin, Am J. Cardiol 56:231-271 (1985); E.G. Nabel et al: Tolerance to long acting nitrates in the treatment of myocardial ischemia out of hospital. Clin Research 34:2 (1986), p.32.

Generally, it is pointed out that some drug free period may help to reduce the incidence and degree of tolerance to nitrates. It is suggested to remove a 24 hour transdermal nitrate delivery device some hours prior to applying a new device. This technique has the disadvantage of being disruptive to patient convenience and compliance which a once a day device provides.

When a transdermal delivery device is applied to the skin there is normally an initial time period which must elapse before the agent is delivered into the body at the predetermined administration rate. This "lag-time" is attributable to various factors including the time required to establish a steady state concentration gradient across the skin and the extent to which the agent is bound or metabolized within the skin. Although the lag-time varies widely from agent-to-agent, it is relatively constant on an individual-to-individual basis for any specific agent and any specific skin site. If agent delivery at therapeutic rates is to be delayed for some time beyond the lag-time for a single application, the skin contacting surface of a transdermal delivery device should be substantially free of available agent when applied to the skin and means should be provided by which the predetermined delay can be obtained with a reasonable degree of reproducibility. For sequential application there may be no lag-time after the initial application because, in many cases, the agent is eliminated by the body from the old site of application at approximately the same rate that it is being deposited at the fresh site of application. Thus if interruptions in administration are to be obtained from sequential administration a similar requirement for an agent-free surface exists.

An osmotic transdermal delivery device which is capable of producing a predetermined delay is disclosed and claimed in co-pending, co-assigned U.S. Patent Application of Theeuwes, et al for Fluid Imbibing Pump With Self-Regulating Skin Patch, S.N. 06/853262, April 17, 1986 (Continuation-in member of S.N. 06/761740 filed August 1, 1985 which was a continuation of S.N. 06/450887 filed December 20, 1982, both now abandoned). Diffusional delivery devices which deliver the agent by diffusion from a reservoir are generally simpler and less expensive than osmotic devices and the applicants are unaware of any prior art describing a diffusional delivery device, generally, or a diffusional transdermal delivery device, specifically, having the characteristics required according to our invention.

U.S. Patent 3,923,939 is noted as being of some relevance to this invention in that it discloses a diffusional delivery device in which the agent releasing surface has been treated to decrease the amount of agent initially present to improve the initial kinetics of the device. The purpose for which the device is treated is, however, different from the purposes of this invention and, even after it has been treated, the agent releasing surfaces of the diffusional delivery device of the '939 patent contains agent at the saturation concentration of the agent in the reservoir.

Accordingly, it is an object of the invention to provide a diffusional delivery device which, following its application to the skin or other biological surface, releases therapeutically beneficial agent according to a predetermined pattern having an initial period of specified duration, when the rate of agent release is maintained below a therapeutic rate, followed by a second period, of specified duration, when agent release occurs at the desired therapeutic rates.

A related object is the delivery of agent after a defined delay following installation or application of a diffusional delivery device.

Another object of this invention is to provide a diffusional delivery device which delays the presentation of agent to the biological environment for a specified period of time after installation or application.

SUMMARY OF THE INVENTION

In accomplishing the foregoing and related objects, the invention provides a medical device for delivering agent or drug to a biological environment by diffusion through a barrier disposed between an agent reservoir and the environment, whereby a delay occurs, beyond the normal lag-time after the device is installed, before agent starts to be delivered at the predetermined therapeutically effective rate. The device has at least two members, one containing agent and the other substantially or completely free of agent (hereinafter referred to as the, "agent-free member"). The agent-free delay member is combined with the agent-containing member, either immediately prior to or after installation at the biological delivery site. The site will typically be the skin, but includes any mucosal or epithelial surface, or within an organism as with an implant. After the agent containing member is brought into contact with the agent-free member, agent is delivered to the environment after a delay imposed by the time required for the agent to diffuse into the agent-free member and establish the concentration gradient associated with desired steady-state delivery rate.

The first member has a reservoir of the therapeutic agent which is to be delivered through an agent releasing surface. The second member is at least substantially agent-free and preferably agent-free. It should be recognized the agent-free member may contain small amounts of the agent at levels well below the saturation concentration of the agent in the member without interfering with the operation of the device according to this invention. These amounts may get into the agent-free member as a result of transfer during manufacturing or storage, and such small amounts are contemplated within the term, "agent-free".

The agent-free member may be a unitary or a composite member which is maintained separated from the agent releasing surface of the agent-containing member until the device is activated by combining the two. The agent-free member is sized to occlude all or substantially all of the agent releasing surface of the agent reservoir, whereby the agent must pass into and through the agent-free member in order to reach the surface of the device at which it is released to the biological environment.

The agent-free member thereby impedes the passage of the agent to the releasing surface and results in a delay in the time at which a steady-state delivery rate from the reservoir to the body is established as compared to a device applied to the body without an agent-free member. The actual delay obtained is established by the properties of the agent and the agent free member and the mathematical models by which the delay may be calculated are described in J. Crank, The Mathematics of Diffusion, Second Edition, Clarendon Press, Oxford (1975) particular reference being directed to Chapters 4 and 12. This reference provides a basis for the calculation of the delays obtained according to this invention for single membrane systems as well as for multi-layered systems. For purposes of illustration, however, reasonable approximations, within the limits of accuracy required for practical embodiments of this invention, of the delay obtained according to this invention from constant activity reservoirs without and with rate control are set forth below.

The delay obtained with a constant activity reservoir without rate control by disposing a single lamina agent-free member between the reservoir and the skin is given by the equation:

$$T = L^2 / 6D \quad (1)$$

where T is the time to steady-state delivery, L is the thickness of the agent-free member and D is the diffusivity of the agent in the material from which the agent-free member is made.

The delay obtained from a rate-controlled, constant flux reservoir by disposing a single lamina agent-free member between the rate controlling element of the reservoir and the skin is given by the equation:

$$T = (L \times C) / (6 \times J) \quad (2)$$

where T is the time to steady state delivery, C is the equilibrium solubility of the agent in the agent-free member, L is the thickness of the agent-free member and J is the in vitro steady-state flux of drug from the rate controlled device into an infinite sink.

In accordance with one embodiment of the invention, the agent-free member is an adhesive through which the agent has the ability to diffuse. Adhesive may additionally be selected for its ability to adhere to the skin or mucosal surface. Acrylates have been found to have satisfactory properties, however a variety of adhesive or other materials may be used, through which agents may diffuse according to the formula stated above. Other examples include polyisobutylene and silicone-based adhesives.

In accordance with another embodiment of the invention, the second member is hingedly attached to the first member. A removable protective liner covers exposed surfaces. In accordance with one embodiment, one liner covers the agent releasing surface, and another liner covers the skin contacting surface of the separate member. The material and design of the hinge should be such as to minimize or prevent the diffusive transfer of agent from the reservoir to the second member.

In accordance with yet another aspect of the invention, the device includes a rate-controlling element which controls the rate at which the agent is released into the environment of use. This element may be either combined with the agent-free member or it may be combined with the agent-containing member. If it is combined with the agent-free member, it will perform dual functions in that it will control the release rate of agent and also extend the length of the delay period according to formula (1). If it is combined with the agent-containing member, its effect on the latter function will be reduced because it will be saturated with the agent at the time of combination. The rate-controlling element may be fabricated from a variety of materials as is known to the art (se U.S.Patent 3598122, for example, which is incorporated herein by reference) which include ethylene/vinyl acetate copolymers.

In accordance with a further aspect of the invention, one or both of the first and second members are composed of contacting laminae. The first member includes an agent-impermeable lamina and agent-containing lamina. The second member is substantially or completely free of agent, and includes an adhesive lamina. When the two members are combined, where the agent-free second member contacts the agent-containing second member, agent flows into the adhesive lamina, where it is absorbed producing a delay in agent delivery to the biological absorbing surface. A rate controlling lamina may be interposed between the agent releasing surface of the first member and the adhesive lamina, as discussed above.

In operation, the agent-free member is installed at the biological absorbing site which is usually skin, but which may be any tissue surface through which agent may be absorbed into the body. The agent-containing member is then attached to the agent-free member to activate the combined device. Agent passes through the agent-free member before reaching the biological environment.

In a yet further aspect of the invention, devices in accordance herewith are used to deliver agents in a novel therapeutic pattern, whereby agent-free intervals are obtained while the agent delivery system is in place. A wide variety of agents may be delivered in this manner, including but not limited to peptides, hormones, and organic nitrates. In general, the devices of this invention are particularly suitable for the delivery of any agent whose beneficial effect is improved or enhanced by periodic interruptions in administration or variations in the concentration of the agent in the blood or body tissues.

It has recently been suggested that periods of organic nitrate administration may be alternated with periods of no agent administration, which could lead to a reduction in the development of tolerance to the agent. Such a result would improve the efficacy with which organic nitrates may prevent or minimize the severity of myocardial ischemia. In accordance with the invention, a device as described herein may be used to deliver organic nitrates after a delay, such as between 4 and 18 hours. As a result, a single delivery device may be replaced once daily, facilitating patient compliance, while offering a nitrate-free interval in a regular therapeutic administration regimen. Also as a result, the nitrate-free interval might conveniently be obtained during the ordinary hours of sleep and terminated by the onset of organic nitrate absorption into the patients body at therapeutically effective rates 1-2 hours prior to the patients usual time of arousal from sleep.

BRIEF DESCRIPTION OF THE DRAWINGS

Other aspects of the invention will become apparent after considering several illustrative embodiments taken in conjunction with the drawings in which:

Fig. 1 is a perspective view of a device in accordance with one embodiment of the invention, illustrating the assembly of an agent delivery system by combining an agent-free member and an agent-containing member at the site of administration;

Fig. 2 is a schematic view of the combined elements of Fig. 1, illustrating the elements in an inset detail;

Fig. 3 is a perspective of an alternative embodiment in accordance with the invention, whereby the agent-containing and agent-free members may be connected for ease in handling by the patient;

Fig. 4 is a schematic cross-sectional view through the device of Fig. 3, particularly illustrating the release layers and connecting segment;

Fig. 5 illustrates the theoretical variation of thermodynamic activity obtained when devices of this invention are at steady state after application to the skin.

Fig. 6 is a theoretical plot of cumulative amount of agent released v time, from a device of the invention; and

Fig. 7 is a theoretical plot of release rate v time, from a device of the invention.

DETAILED DESCRIPTION OF THE INVENTION

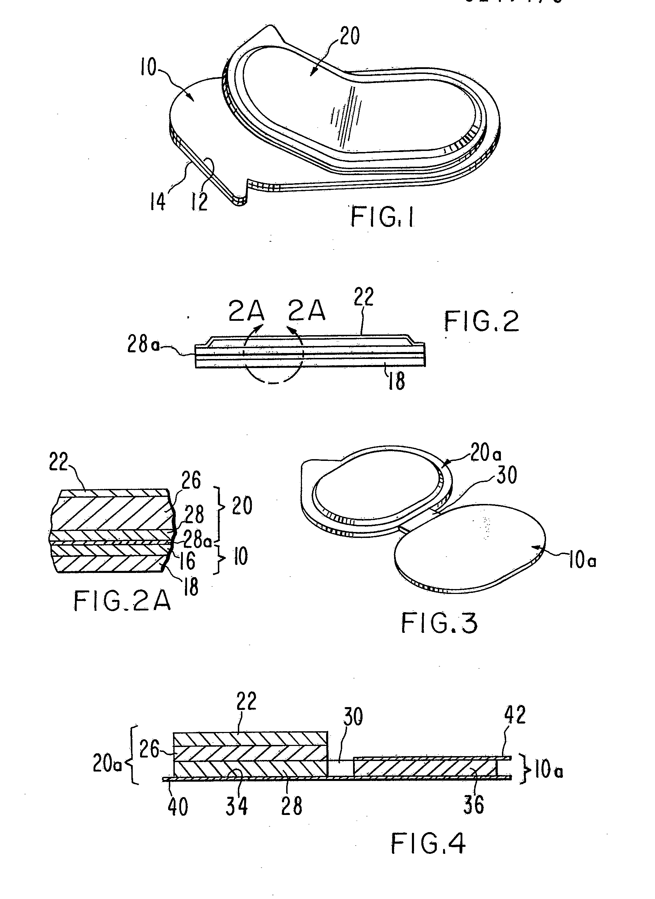

With reference to the Figures, a device 1 according to this invention comprises a member 10 which is substantially devoid of agent until activation by combination with the agent-containing member. An agent-containing member 20 which contains the agent reservoir is applied to member 10 to activate same, whereupon agent is administered through member 10 to the biological environment which in Fig. 1 is shown as the skin. Member 10 is sized and shaped to occlude substantially all of the agent releasing surface of member 20 and functions to delay passage of agent from reservoir 20 to the skin, mucosa or other biological surface.

Members 10 and 20 can take various forms. In the simplest form for transdermal administration member 10 could simply be an agent-free adhesive layer and member 20 could be an agent-containing matrix which may or may not be adhesive. Typically, member 20 would be provided with an agent-impermeable backing to prevent the release of agent from the skin-distal surface of the device. Prior to use, the exposed surfaces of members 10 and 20 could also be provided with removable release liners, not shown, as is known to the art with other drug delivery devices. In other embodiments, one or more elements can be combined with basic members to provide rate control or to improve strength as will be more readily apparent below.

With reference to Fig. 1, a device 1 in accordance with the invention comprises an agent-free member 10 having inner and outer surfaces 12 and 14 respectively. Surface 14 is disposed to lie in conformity with a wearer's skin when the device is installed, and is preferably adhesive in character. Surface 12 confronts the agent-containing member 20, and preferably adheres thereto when combined.

With reference to Fig.2, member 20 comprises a backing layer 22 which is impermeable to the agent disposed within reservoir 26. If the material in which agent is dispersed to form the reservoir 26 is not fluid at temperatures to which it is likely to be exposed, the edges of reservoir 26 need not be sealed. As shown, backing layer 22 is sealed about its periphery to layer 28 to fully enclose reservoir 26. In some embodiments of the invention layer 28 may perform a release-rate controlling function, it may perform a strengthening or reinforcing function and it may or may not an adhesive. If layer 28 is not adhesive, an adhesive coating layer 28a may optionally be provided if required to adhere to a non-adhesive surface of member 10.

Member 10 may comprise a layer 16 which may perform functions similar to those performed by layer 28 although in typical embodiments only one of layers 28 or 16, if present, would be release-rate controlling layers. When members 10 and 20 are combined at the time of application, agent passes from the reservoir 26 through the agent releasing surface of member 20 and into agent-free member 10.

In accordance with the invention, it is desired to delay the passage of agent from the device for a period of hours after the members 10 and 20 are combined. In order to achieve this, agent must not pass too quickly through member 10, in order that a lag of beneficial length may be obtained. Advantageously, a steady state of agent delivery is obtained after the delay period has ended. The elements comprising members 10 and 20 can be combined in a variety of configurations perform these functions.

In one version of Fig. 2. member 10 contains a non-adhesive layer 16 which functions as a rate-controlling membrane and layer 28 of member 20 is highly permeable to the agent to be delivered and functions to seal reservoir 26 between backing layer 22 and to act as a substrate for thin adhesive coating 28a. Examples of suitable rate-controlling materials may be found in the cited references and related art. An example is a 2 mil thick membrane of ethylene/ vinyl acetate copolymer, of 9% vinyl acetate. Layer 18 is an adhesive which cooperates with layer 16 to provide for the desired delay in administration of the agent at therapeutic rates after activation. Suitable adhesives include mixtures of high and low molecular weight polyisobutylenes and acrylate or silicone based adhesives. Additional examples of adhesives may be found in the cited references, which are non-irritating to the skin, adhere under moist conditions, and provide a pathway for agent diffusion.

In Figs. 3 and 4, an alternative embodiment of the invention is shown, wherein an agent containing member 20a is joined to a delay member 10a by a connecting segment 30. In this embodiment, members 10a and 20a are folded to confront and mutually adhere surfaces 34 and 36. Segment 30 encourages the proper alignment of members 10a, 20a. In order to prevent agent from migrating from member 20a to member 10a during storage, for example, member 30 may be made from a material substantially impermeable to the agent being delivered.

In Figure 4 an embodiment of the invention which does not fully enclose the reservoir 26 is shown. In this embodiment layer 26 is an agent loaded adhesive containing dispersed agent, typically at a loading sufficiently above saturation to provide an adequate supply of agent to maintain unit thermodynamic activity throughout the administration period. Layer 28 may or may not perform a rate-controlling function and member 10a comprises an agent-free adhesive disposed between removable release liners 40 and 42. Adhesive 36 may or may not perform a rate-controlling function.

As can be seen in Fig.4, a releasable liner 40 extends across the contacting surfaces of members 10a and 20a. Another liner 42 covers the skin contacting surface of member 10a. Liners 40 and 42 may be fabricated from siliconized paper, for example, whereby they are peeled off without removing or damaging the adhesive layer to which they are adhered. Where member 10a is not connected to member 20a by segment 30, liner 40 has a lower peel strength than liner 42. Thus, liner 42 is removed, member 10a is attached to the skin, liner 40 is removed, and member 20a is attached to the surface of member 10a thus exposed. Hinge 30 is preferably formed from a flexible, agent impermeable material which is bonded or otherwise affixed at its sides to layer 28 and member 10a.

Members 10 or 10a impose a delay on the passage of agent from member 20 or 20a. The time required for an agent to diffuse through the barrier formed by members 10 or 10a may be determined from formula (1) as described above.

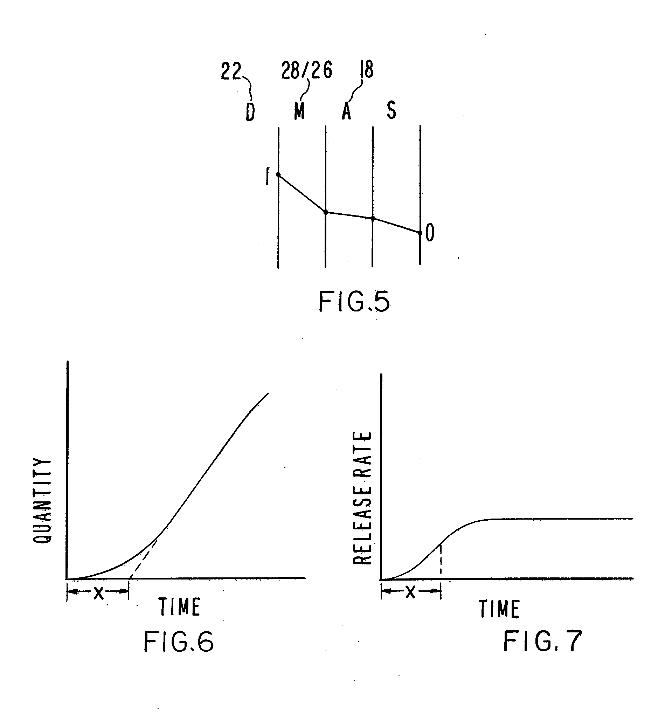

Figure 5 illustrates the theoretical change in thermodynamic activity of the agent across the various layers of an embodiment of this invention and the skin at the steady state achieved after application to the skin. For illustrative purposes all layers are shown as having the same thickness, it being recognized that in an actual embodiment the thickness of the various layers will be determined according to the function they are to perform and the nature of the materials and the agent being delivered. In Fig. 4, the layers are agent reservoir D, rate controlling membrane M, adhesive layer A, and the skin S. The agent reservoir D is considered to have unit activity, and the site of agent absorption at the inner surface of the skin S is assumed to be an infinite sink having a thermodynamic activity of zero. The slope of the line through each layer represents the change in the thermodynamic activity that occurs across each layer. Thus, for layers of assumed equal thickness, steeper slopes indicate higher resistance to mass transport. The layer having the steepest slope is the layer that acts as the rate controlling element of the combined device-skin system. In order for the device, rather than the skin, to exert a preponderance of rate control, the slope of the line across a layer of the device should be greater than the slope of the line across the skin.

The materials used to form the agent-free member can therefore be selected to perform one or more functions. If the agent-free member is intended to produce a delay without imposing any significant rate-control, the material should have a low diffusivity and a high permeability to the agent (Permeability is the product of the diffusivity and the saturation concentration of the agent in the material forming the agent-free member). If the agent-free member is to produce a delay and impose significant rate control, the material should exhibit both a low diffusivity and a low permeability.

As illustrated in Fig. 5, layer M has the steepest slope and the device would be considered a rate-controlled device. It should also be noted that, as illustrated, the slope across the adhesive layer is slightly steeper than across the skin and thus the adhesive layer also imposes some rate control. Adhesives are generally highly soluble to agents, yet may be provided in sufficient thickness to exhibit greater rate controlling characteristics. As the components forming member 10 or 10a are made thicker, increasing delays are produced because more agent must be introduced into the initially agent-free member before its capacity and the capacity of the skin to receive agent is reached and member 10 or 10a begins to deliver agent into and through the skin at the steady-state rate ultimately intended.

Fig. 5 illustrates the conditions in existence during steady-state agent delivery. At the moment of assembly of the agent-free and agent-containing members and application to the skin, the conditions are quite different. The activity throughout the layers comprising the agent-containing member will be at unit activity up to the agent releasing surface thereof. The activity across the layers comprising the agent-free member and the skin will, however, be zero. The delay in on-set of agent delivery at the steady-state rate is the result of the time required for the agent to diffuse from the reservoir through the interface between the agent-containing member and the agent-free member to establish the activity gradients shown in Fig. 5.

Prior to attainment of steady-state delivery some agent will be delivered at pre-steady state rates. These rates will vary with time from zero to the steady-state rate and normally will be below therapeutic rates for a substantial portion of the initial time period. This substantial portion of the time between application and achievement of steady-state delivery provides the desired agent-free interval.

Having thus generally described our invention, the following example illustrates how this invention may be practiced with a commercially available rate controlled transdermal delivery device to produce predetermined delays in the onset of therapeutic effect.

EXAMPLE 1

In this example member 20, which is to be combined with agent-free member 10, is the Transderm-Nitro® nitroglycerin delivering adhesive skin patch sold by Ciba-Geigy Corporation. For that system, an agent-free member device can be designed to produce the desired delay according to this invention, as follows. A Transderm-Nitro delivery device has a nominal in vitro delivery rate of 40 mcg/hr/cm$^2$ and a nominal in vivo delivery rate through intact skin of approximately 20 mcg/hr/cm$^2$. For such a system, in order to produce a delay without significantly affecting the steady-state in vivo flux the delay membrane would be selected to have a high solubility to nitroglycerine in order to have the characteristics of low diffusivity and high permeability described above. Typical silicone medical adhesives such as that use in the Transderm-Nitro system have a nitroglycerine solubility of about 50,000 mcg/cm$^3$. The thickness of a film of such material required to produce the desired delay with this rate controlled system can be calculated from rearrangement of Equation (2) as follows:

$$L = 6(T \times J)/ C \quad (3)$$

Substitution of the values for solubility, in vitro flux and desired delay time into Equation (3) and solving for L results in the required thickness of a silicone adhesive agent-free membrane. In order to produce a delay of approximately 8 hours a membrane of approximately 0.02 cm thick is required. A delay of about 16 hours could be obtained from a similar membrane approximately 0.04 cm thick. This film may be provided to a patient sandwiched between release liners and in a configuration slightly larger than that of the Transderm-Nitro system with which it is to be used. Alternatively the membrane could be hingedly attached to the system with which it is to be used by an impermeable, flexible release liner as described above.

Theoretical in vivo delivery patterns which are obtainable according to the embodiment of Example 1 in terms of cumulative quantity of agent delivered v time and delivery rate v time are illustrated in Figs. 7 and 8, respectively. X represents the delay in steady state administration obtained according to this invention.

Devices in accordance with the invention are particularly suitable for the transdermal administration of therapeutic agents although their application is not limited that mode of delivery. Similarly , the invention is not limited to any specific agent and is applicable to a variety of agents, particularly those which produce a tolerance, such as nitroglycerine or those whose therapeutic effect is to be delayed from a convenient time of application to an inconvenient time of onset such as with nitroglycerin or anti-Parkinsonism drugs. In the latter cases application at bedtime may be convenient, for example, and onset of therapeutic effect may be desired shortly before arousal.

In certain therapeutic situations it has been suggested that the effectiveness of an agent can be improved if periods of therapeutic blood levels are interrupted by agent-free periods. A variety of materials may be advantageously delivered in this manner, including, without limitation, corticosteroids, peptides, and nitrates. Delayed onset or interrupted administration of agents has potential application with all agents whose pharmacodynamic properties show their beneficial actions to be substantially enhanced by periodic intervals in which the agent is absent from the body altogether or substantially so, or present at substantially reduced concentrations in blood or other body fluids.

In the treatment of myocardial ischemia, for example, it has been suggested to interrupt, for varying time periods, the continuous transdermal administration of nitrates such as isosorbide dinitrate or nitroglycerin to prevent or minimize the development of tolerance to the therapeutic effect of the agent. There has been suggestion in the prior art to remove a transdermal delivery device and apply another device after a predetermined interval has elapsed. Intervals varying from 1.5 to 12 hours during daily therapy have been suggested.

One of the advantages of chronic transdermal therapy is the improvement in patient compliance that is obtained the inherent simplicity of changing devices at the same convenient time every day one time. This advantage is lost when removal and application occur at different times or where onset of a therapeutic effect is desired at an inconvenient time such as during sleep. The devices of this invention are particularly useful in providing a predetermined, delayed onset of therapeutic effect for any desired time period after application to the skin. Thus, a device could be removed and a new one applied concurrently, wherein the desired agent-free interval is obtained. For example, it is possible according to this invention to change devices such as described in Example 1 at bedtime and have the nitrate free interval occur between midnight and 5 AM, when myocardial ischemic attacks are usually fewest and least severe, with full therapeutic concentrations of nitrate re-established by the time of awakening at e.g. 7 AM, when ischemic attacks are usually most numerous and most severe during the 24-hour cycle.

The therapeutic problem of nitrate tolerance has received increasing attention. It is believed that the agent-free interval may serve as a remedy for tolerance, tachyphylaxis, and receptor fade/down-regulation. Tolerance is particularly indicated where the strength of beneficial action wanes over time. As stated by Jonathan Abrams in THE AMERICAN JOURNAL OF CARDIOLOGY, VOL 56, JULY 10, 1985, P17A:

Some have suggested that formulations providing sustained nitrate plasma concentrations to blood vessels or repeated administration of large doses of nitrates may induce tolerance more readily than interrupted or lower dose therapy. This may be particularly relevant to the transdermal NTG discs. There is tantalizing clinical evidence to support these theories but the final answer is far from in. If true, pushing nitrate dosage to maximally acceptable amounts could actually exacerbate the development of drug tolerance. It appears likely that interrupted therapy is less likely to cause vascular tolerance...

...Tolerance should be suspected when a patient has initially achieved a satisfactory response to nitrate compounds, but then finds chest pain becoming more frequent or more protracted. Obviously other possibilities can explain this phenomenon, including worsening of the underlying disease state or other aggravating factors triggering more frequent episodes of angina. Nevertheless, if it is felt that nitrate tolerance could be the cause of the clinical deterioration, the alert physician should either discontinue nitrates for a short period of time or increase the dose, or both. Parker et. al. have shown that halting nitrates for as little as 18 hours restores vascular sensitivity to the actions of nitroglycerin.

Although the prior art suggests a wide range of nitrate free intervals, it is the inventor's belief that nitrate delivery / no-delivery periods, in hours, of 12/12 to 20/4 are most likely to yield improved anti-ischemic results, compared to continuous administration around-the-clock. Thus delivery profiles of 12/12, 16/8 and 20/4 may be indicated for nitrate therapy, and are attainable with devices in accordance with the invention. The present invention achieves such profiles by imposing a delay period upon the application of a transdermal device in accordance therewith. Thus, to achieve a pattern of approximately 16 hours of delivery followed by approximately 8 hours of non-delivery, a device according to this invention should have a delay of approximately 8 hours before delivery at therapeutic rates is commenced. It should thereafter continue to deliver agent at the therapeutic rate for the next 16 hours, at which time it is replaced with a fresh device which begins its duty cycle with an approximately 8 hour period during which no, or substantially no, agent is delivered at therapeutic rates.

Alternatively, a 24 hour device may have a delivery duration of 16 hours with a 4 hour delay. Agent delivery would thus taper off 4 hours before a replacement patch is applied. This latter model, in which agent free intervals are provided at both the beginning and end of the duty cycle is not as predictable as the first model. This is because it is much more difficult to predict with precision precisely how long it will take to deliver all the contained agent for all individuals. With the first model, however, the device is merely provided with sufficient agent to ensure delivery for 16 hours in all wearers. For either model, device are changed each day at the same time, as is the current practice in a continuous mode of transdermal therapy.

In achieving an agent-free interval from a diffusional agent delivery device, it is desirable that the amount of diffusionally available agent at the agent releasing surface be either zero or at a low level compared to the concentration that is established at the steady-state rate. The lower the initial concentration of the agent in the agent-free member, the more effective the device is in achieving the desired delay. As discussed above, prior art diffusional delivery devices do not achieve this condition because their agent releasing surfaces are at the equilibrium concentration of the agent in the material from which the surface is formed and which as noted above, if the material is an adhesive, can be quite high. The invention maintains the agent reservoir in non-agent-transmitting relation to the element of the device through which agent is released to the body until the device is activated by assembly of the separate elements and installation at the delivery site. Thereafter agent cannot be delivered at therapeutic rates until the agent diffuses into and through the agent-free elements and the body surface separating the device from the infinite sink of the bloodstream and establishes a thermodynamic activity gradient across which the agent may diffuse at the desired rates. Thus by appropriate selection of the composition and thickness of the elements comprising the agent-free portions of the device, great flexibility in the length of the predetermined delay period can be attained.

## Claims

1. An active agent delivery device for delaying the administration of an active agent to the body for a period of time, T (hr), of at least about 4 hours after application of the device at the delivery site, said device having a therapeutically effective steady state in vitro flux, J ($\mu$g/cm$^2$ hr) comprising, in combination:

(1) active agent reservoir means having an active agent releasing surface through which active agent is released by diffusion;

(2) delay means comprising a substantially active agent-free, active agent permeable material having a predetermined active agent solubility C ($\mu$g/cm$^2$ hr), and a thickness, L (cm), said delay means being physically separate from, and being sized to occlude substantially all of said active agent releasing surface; and

(3) means for placing said reservoir means and said delay means in active agent transmitting relationship to the administration site; the relationship between L, T, J and C being given by the equation:

$$L = 6(T \times J)/C$$

whereby said reservoir and said delay means may be combined concurrently with application at the administration site to produce a predetermined delay of at least about 4 hours in the onset of active agent delivery at therapeutically effective rates.

2. A device as claimed in claim 1 wherein the delivery site is an area of skin and means (3) comprises a contact adhesive disposed between the agent releasing surface of reservoir means (1) and the skin.

3. A device as claimed in claim 2 wherein said delay means (2) comprises, at least in part, said adhesive.

4. A device as claimed in any preceding claim further comprising agent impermeable connecting means hingedly connecting said delay means to said reservoir means.

5. A device as claimed in any preceding claim wherein said agent is selected from the group consisting of corticosteroids, peptides and nitrates.

6. A device as claimed in any preceding claim wherein said device further comprises release rate controlling means which maintains the steady state in vitro flux (J) substantially constant during a predetermined agent administration period.

7. A device as claimed in any preceding claim wherein said agent is nitroglycerin.

8. A device as claimed in claim 7 wherein further comprising nitroglycerin release rate controlling means which limit the steady state in vitro flux to no greater than about 40 $\mu$g/cm$^2$ hr of nitroglycerin; and said delay means comprises a substantially nitroglycerin-free, nitroglycerin permeable material having a solubility for nitroglycerin of approximately 50,000 $\mu$g/cm$^3$ and a thickness of at least about 0.01 cm.

9. A device as claimed in any preceding claim wherein said adhesive is selected from polyisobutylene, acrylate and silicone adhesives.

10. A device as claimed in any one of claims 6 to 9 wherein said release rate controlling means forms a part of said reservoir means.

0249475

FIG.1

FIG.2

FIG.2A

FIG.3

FIG.4

0249475

FIG.5

FIG.6

FIG.7